# EUROPEAN PATENT APPLICATION

(11) **EP 4 821 525 A1**
(43) Date of publication of application: **14.10.2026**
(21) Application number: 24900803.8
(22) Date of filing: 27.09.2024
(51) Int. Cl.: A61M 15/00

(54) **INHALER**

(30) Priority: 07.12.2023 KR 20230177003
(71) Applicant: KT&G Corporation, Daedeok-gu Daejeon 34337 (KR)
(72) Inventor: KIM, Moonwon, Daejeon 34187 (KR); SEO, Man Seok, Seoul 05387 (KR); JANG, Yong Joon, Daejeon 34071 (KR); JUNG, Yongmi, Sejong 30153 (KR); SHIN, Jun Won, Sejong 30098 (KR)
(74) Representative: Ter Meer und Partner mbB
(86) International application number: PCT/KR2024/014691
(87) International publication number: WO 2025/121615

(57) **Abstract**

An inhaler according to an embodiment of the present invention comprises: a housing which comprises a first surface, a second surface facing the first surface, and a side surface connecting the first and second surfaces, and which is provided with a plurality of air inlets penetrating the side surface in the vertical direction; a mouthpiece protruding from the first surface; and a guide forming a path through which the air flowing in from the air inlets moves inside the housing, wherein the guide can guide functional material accommodated inside the housing to the mouthpiece when suction force is applied thereto.

## Description

### Technical Field

Methods and apparatuses consistent with embodiments relate to an inhaler.

### Background Art

A conventional dry powder inhaler is intended to deliver a single-dose, metered dose of a pharmacological agent into the body and has been used primarily to treat patients with asthma and chronic obstructive pulmonary disease (COPD). Finely dried powders of 5 µm to 10 µm or less are used for efficient absorption of the agent into the body. In addition, a determined amount of drug may be filled into a hard capsule for quantitative delivery.

A dry powder inhaler may consist of a capsule filled with dry powder and an inhalation device. Technology for manufacturing dry powder and technology for designing a device that generates a vortex for delivering dry powder may be required.

The above description is information the inventor(s) acquired during the course of conceiving the present disclosure, or already possessed at the time, and is not necessarily art publicly known before the present application was filed.

### Disclosure of the Invention

### Technical Problem

Embodiments provide an inhaler that is capable of performing transfer of dry powder to the body by inhalation pressure after simple attachment or filling without a process of crushing or perforating an inhalation capsule filled with dry powder, through a dedicated cartridge or a direct powder filling process, and that is capable of inducing transfer of powder through upward airflow caused by inhalation, by utilizing an internal structure of the inhaler.

The technical goals obtainable from the embodiments are not limited to the above-mentioned technical goals, and other unmentioned technical goals may be clearly understood from the following description by one of ordinary skill in the art to which the present disclosure pertains.

### Solution to Problem

According to an aspect of an embodiment, there is provided an inhaler including a housing including a first surface, a second surface opposite to the first surface, and a side surface configured to connect the first surface to the second surface, wherein a plurality of air inlets is formed to penetrate the side surface in a vertical direction, a mouthpiece protruding from the first surface, and a guide configured to form a path through which air introduced through the air inlets moves in an interior of the housing, wherein, when an inhaling force is applied to the mouthpiece, the guide is configured to guide a functional material accommodated in the interior of the housing to be transferred to the mouthpiece.

According to another aspect of an embodiment, there is provided an inhaler including a housing including a first surface, a second surface opposite to the first surface, and a side surface configured to connect the first surface to the second surface, wherein a functional material is accommodated in an interior of the housing, a mouthpiece protruding from the first surface, and an airflow path formed in the interior of the housing and extending from a plurality of air inlets, which is configured to penetrate the side surface in a vertical direction, to the mouthpiece, wherein, when an inhaling force is applied to the mouthpiece, the functional material is transferred to the mouthpiece along the airflow path together with air introduced through the air inlets.

### Advantageous Effects of Invention

According to embodiments, an inhaler may perform transfer of dry powder to the body by inhalation pressure after simple attachment or filling without a process of crushing or perforating an inhalation capsule filled with dry powder, through a dedicated cartridge or a direct powder filling process, and may induce transfer of powder through upward airflow caused by inhalation, by utilizing an internal structure of the inhaler.

The effects of the inhaler are not limited to the above-mentioned effects, and other unmentioned effects can be clearly understood from the following description by one of ordinary skill in the art to which the present disclosure pertains.

### Brief Description of Drawings

FIG. 1 is a diagram illustrating an inhaler according to an embodiment;
FIG. 2 is a diagram illustrating an internal structure of an inhaler, according to an embodiment;
FIG. 3 is a diagram illustrating internal airflow in an inhaler, according to an embodiment;
FIG. 4 is a diagram illustrating a cartridge of an inhaler, according to an embodiment.

The accompanying drawings illustrate desired embodiments of the present disclosure and are provided together with the detailed description for better understanding of the technical idea of the present disclosure. Therefore, the present disclosure should not be construed as being limited to the embodiments set forth in the drawings.

### Best Mode for Carrying Out the Invention

Hereinafter, embodiments are described in detail with reference to the illustrative drawings. Regarding the reference numerals assigned to the components in the drawings, it should be noted that the same components are designated by the same reference numerals, wherever possible, even though they are shown in different drawings. Furthermore, in the description of the embodiments, detailed description of well-known related configurations or functions is omitted when it is deemed that the description will cause ambiguous interpretation of the embodiments.

In addition, in the description of the components of the embodiments, terms such as first, second, A, B, (a), (b), and the like may be used. These terms are used only for the purpose of discriminating one component from another component, and the nature, the sequences, the orders, or the like of the components are not limited by the terms. It is to be understood that if a component is described as being "connected," "coupled," or "joined" to another component, the former may be directly "connected," "coupled," or "joined" to the latter or "connected," "coupled," or "joined" to the latter via another component.

The same name may be used to describe components having a common function in different embodiments. Unless otherwise mentioned, the description of one embodiment may be applicable to another embodiment. Thus, duplicated description is omitted for conciseness.

The present disclosure relates to an inhaler 10 for inhaling a functional material provided as dry powder, the inhaler 10 that allows inhaling dry powder without a separate inhalation capsule filled with dry powder.

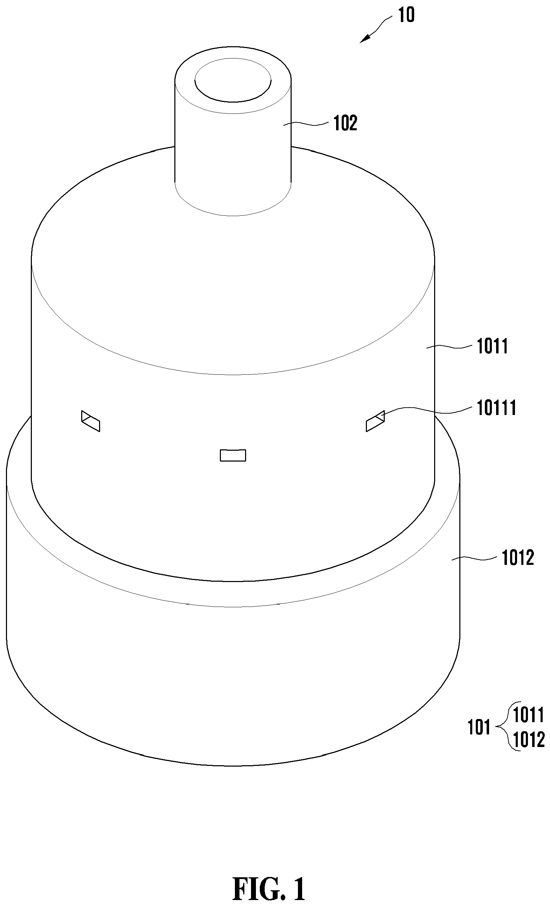

FIG. 1 is a diagram illustrating the inhaler 10 according to an embodiment.

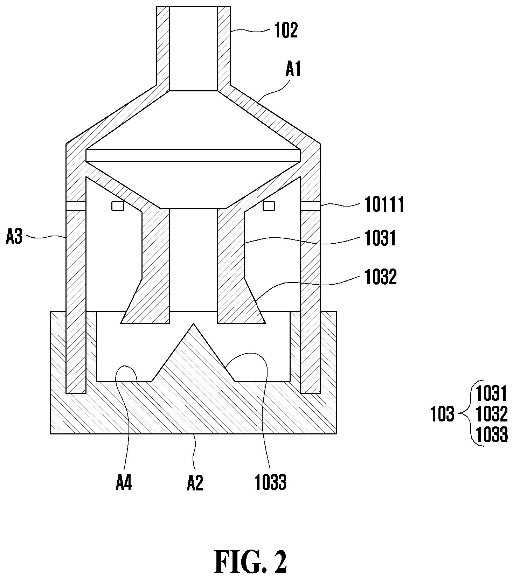

FIG. 2 is a diagram illustrating an internal structure of the inhaler 10, according to an embodiment.

Referring to FIG. 1, the inhaler 10 may include a housing 101, a mouthpiece 102, and a guide 103.

The housing 101 may include a first surface A1, a second surface A2 opposite to the first surface A1, and a side surface A3 connecting the first surface A1 to the second surface A2.

A plurality of air inlets 10111 may be formed in the housing 101. The air inlets 10111 may be formed to penetrate the side surface A3 in a vertical direction.

A functional material may be accommodated in an interior of the housing 101. The functional material may be accommodated at a position adjacent to the second surface A2. The functional material may be provided in a powder form.

For example, the housing 101 may include a holder 1011 and a cartridge 1012.

The holder 1011 may include the first surface A1 and may be connected to the mouthpiece 102 at the first surface A1. The holder 1011 may extend from the first surface A1 toward the second surface A2. For example, the holder 1011 may be formed in a cylindrical shape.

The holder 1011 may be of a size suitable for a user to hold by the hand. For example, the holder 1011 may have a diameter dimension of less than 50 mm for this purpose. Preferably, the diameter dimension may be around 20 mm to 30 mm.

The holder 1011 may have the air inlets 10111 formed on the side surface A3. Air may be introduced from the outside through the air inlets 10111. The air inlets 10111 may be provided in plurality. Each of the air inlets 10111 may be disposed to be spaced apart from another in a circumferential direction of the side surface A3. Each of the air inlets 10111 may be formed in a direction toward a center of a holder 1011 to generate a laminar flow or a vortex flow in an interior of the inhaler 10. In addition, the air inlets 10111 may be formed to be positioned tangentially in order to generate a rotational force in the airflow. For example, a cyclone effect may be induced by the air inlets 10111 arranged in this manner.

Each of the air inlets 10111 may be provided with an opening and closing member. The opening and closing member may operate to close the air inlets 10111 when the user is not using the inhaler 10 and to open the air inlets 10111 when the user is using the inhaler 10.

In addition, a space through which the airflow moves may be formed in an interior of the holder 1011. Introduced air may move in the interior of the housing 101 through the space.

The cartridge 1012 may include the second surface A2 and may be detachably coupled to a lower portion of the holder 1011. The cartridge 1012 may accommodate the functional material therein. When all of the accommodated functional material is exhausted, a user may replace the cartridge 1012 with a new cartridge 1012 or may separate the cartridge 1012 from the holder 1011, fill an interior of the cartridge 1012 with the functional material, and reattach the cartridge 1012 to the holder 1011.

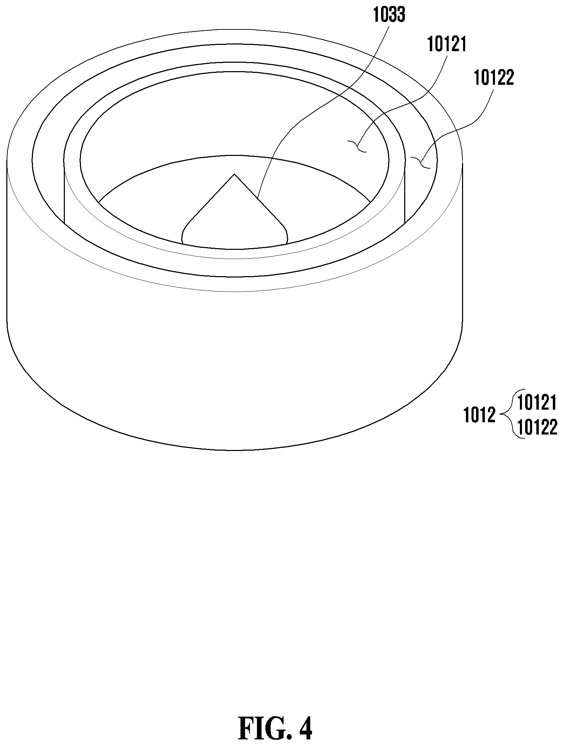

The cartridge 1012 is described in more detail below with reference to FIG. 4.

The mouthpiece 102 may be formed on the first surface A1 of the housing 101. The mouthpiece 102 may protrude in a direction from the second surface A2 toward the first surface A1.

When an inhaling force is applied to the mouthpiece 102, air may be introduced through the air inlets 10111. The mouthpiece 102 may be provided with a valve. The valve may operate to close an opening of the mouthpiece 102 when the user is not using the inhaler 10 and to open the opening of the mouthpiece 102 when the user is using the inhaler 10. The valve may be provided as, for example, a one-way valve. Accordingly, a residue or an external contaminant may be prevented from being reintroduced into the inhaler 10.

The guide 103 may form a path through which air introduced from the air inlets 10111 moves in the interior of the housing 101.

When an inhaling force is applied to the mouthpiece 102, the guide 103 may guide the air introduced through the air inlets 10111 to move toward the second surface A2 to reach the functional material and may guide the functional material to be transferred toward the mouthpiece 102 together with the air.

Hereinafter, the guide 103 is described in more detail with reference to FIG. 2.

Referring to FIG. 2, the guide 103 may include a first guide 1031, a second guide 1032, and a third guide 1033. The guide 103 may form an airflow path 104 through which introduced air and a functional material may move.

The first guide 1031 may extend from a height adjacent to the air inlets 10111 in a direction from the first surface A1 toward the second surface A2. The first guide 1031 may extend in a direction perpendicular to the air inlets 10111. This first guide 1031 may guide air introduced through the air inlets 10111 to move toward the second surface A2. The first guide 1031 may form a first path 1041.

The second guide 1032 may extend from the first guide 1031 toward the second surface A2. In this case, the second guide 1032 may extend from the first guide 1031 in a flange shape. An end portion of the second guide 1032 may be spaced apart from a bottom surface A4. Accordingly, a velocity of the air may increase as the air passes through the second guide 1032. The second guide 1032 may form a second path 1042.

A hollow extending coaxially with the mouthpiece 102 may be formed in interiors of the first guide 1031 and the second guide 1032. A space of the hollow may form a fourth path 1044.

The third guide 1033 may be disposed to be spaced apart from the second guide 1032. For example, the third guide 1033 may be formed on the bottom surface A4 of the cartridge 1012. The third guide 1033 may protrude from the bottom surface A4 in a direction toward the first surface A1. The third guide 1033 may extend toward an axis of the mouthpiece 102.

For example, the third guide 1033 may be formed in a shape in which a cross-sectional area decreases in a direction from the second surface A2 toward the first surface A1. For example, the third guide 1033 may be formed in a cone shape with a diameter that decreases in the direction from the second surface A2 toward the first surface A1. The third guide 1033 may form a third path 1043.

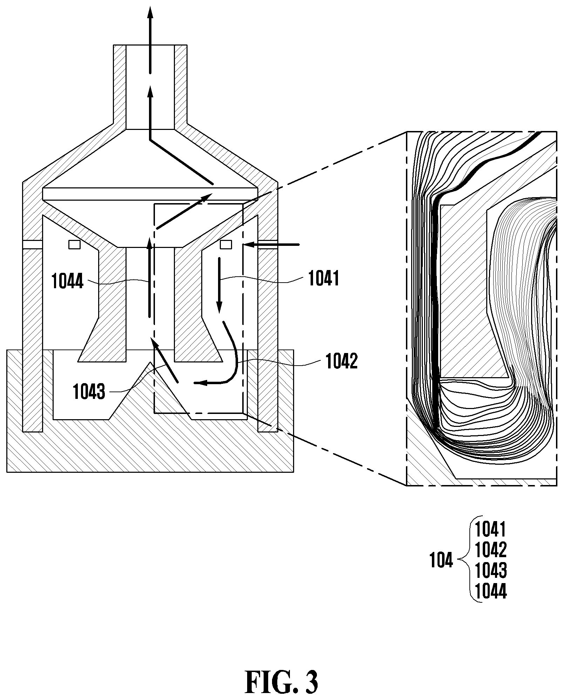

Hereinafter, the airflow path 104 formed by a structure of the guide 103 is described in more detail with reference to FIG. 3.

FIG. 3 is a diagram illustrating internal airflow in the inhaler 10, according to an embodiment.

By the structure of the guide 103 described above, the airflow path 104 having a shape illustrated in FIG. 3 may be formed in the interior of the housing 101.

Referring to FIG. 3, the airflow path 104 may extend from the air inlets 10111 to the mouthpiece 102 and connect the air inlets 10111 to the mouthpiece 102. When an inhaling force is applied to the mouthpiece 102, the functional material may be transferred to the mouthpiece 102 along the airflow path 104 together with air introduced through the air inlets 10111.

For example, the airflow path 104 may include a first path 1041, a second path 1042, a third path 1043, and a fourth path 1044.

The first path 1041 may be formed in a direction perpendicular to the air inlets 10111 and may extend from the air inlets 10111 toward the second surface A2. The first path 1041 may be an airflow path formed by the first guide 1031. The air introduced through the air inlets 10111 may move along the first path 1041 in a direction from the first surface A1 to the second surface A2.

The second path 1042 may extend from the first path 1041 to the bottom surface A4 on which the functional material is accommodated. The second path 1042 may be a path formed by the second guide 1032. The air that moves along the first path 1041 may move along the second path 1042 to the bottom surface A4 to come into contact with the functional material and may carry the functional material toward the third path 1043.

The third path 1043 may extend diagonally from the second path 1042 toward an axis of the mouthpiece 102. The third path 1043 may be formed by a conical structure of the third guide 1033. The air that carries the functional material may rise toward the fourth path 1044 while moving along the third path 1043 in line with the axis of the mouthpiece 102.

The fourth path 1044 may extend from the third path 1043 toward the first surface A1. The fourth path 1044 may extend coaxially with the mouthpiece 102. The fourth path 1044 may be formed by the first guide 1031 and the second guide 1032. When reaching the fourth path 1044 along the third path 1043, the functional material may rise vertically toward the mouthpiece 102 along the fourth path 1044.

In addition, a fifth path may be formed between the mouthpiece 102 and the fourth path 1044.

The fifth path may be formed in a shape in which a diameter increases and decreases again in a direction from the fourth path 1044 toward the mouthpiece 102. The fifth path may be formed, for example, in a diamond structure. The fifth path may prevent the functional material from clumping as the space extends from the fourth path 1044. Since the functional material may evenly spread within the fifth path before passing through the mouthpiece 102, a user may smoothly inhale the functional material when the functional material passes through the mouthpiece 102.

As described above, the functional material may be transferred toward the mouthpiece 102 by the air introduced through the air inlets 10111 and pass through the mouthpiece 102 to be inhaled by the user.

Referring to the enlarged view of FIG. 3, airflow may be introduced from the air inlets 10111, diffuse in the first path 1041, and descend in a direction toward the second surface A2. Thereafter, a flow velocity may increase as a cross-sectional area of the second path 1042 gradually decreases, and the path may change into a curved shape as the airflow comes into contact with the bottom surface A4 on which the functional material is positioned. The airflow moving from the side surface A3 to a center portion due to a shape of the third path 1043 may move upward in a coaxial direction with the mouthpiece 102 while carrying the functional material. The airflow may rise vertically and coaxially with the mouthpiece 102 while passing through the fourth path 1044. The flow velocity may decrease again as the airflow diffuses when reaching the fifth path, and the airflow may carry the functional material to the user while passing through the mouthpiece 102.

Hereinafter, the cartridge 1012 is described in more detail with reference to FIG. 4.

FIG. 4 is a diagram illustrating the cartridge 1012 of the inhaler 10, according to an embodiment. For example, the cartridge 1012 may be formed in a cup shape.

Referring to FIG. 4, the cartridge 1012 may include an accommodation portion 10121 and a coupling portion 10122.

The accommodation portion 10121 may have one side formed as an open and concave space. The functional material may be accommodated in the space. When the cartridge 1012 is coupled to the holder 1011, the concave space formed in the accommodation portion 10121 may communicate with a space which is formed in the holder 1011 and through which airflow may move. Thus, the concave space of the accommodation portion 10121 and the space of the holder 1011 through which airflow may move may together form an internal space of the housing 101. In addition, when the holder 1011 and the cartridge 1012 are integrally formed, the housing 101 may have an integral internal space which may accommodate a functional material and through which airflow may move. In this case, the bottom surface A4 may be defined as a bottom surface of the internal space of the housing 101. The third guide 1033 may be disposed at a center of the accommodation portion 10121. The third guide 1033 may protrude from the bottom surface A4 in a direction toward the first surface A1. The functional material may move to rise upward toward the axis of the mouthpiece 102 along a shape of the third guide 1033.

The coupling portion 10122 may be disposed to be spaced apart in an outer radial direction from the accommodation portion 10121. The coupling portion 10122 may be formed as a groove. A depth of the groove may be formed so that the groove may be closer to the second surface A2 than to the bottom surface A4. A lower portion of the holder 1011 may be inserted into the coupling portion 10122. In addition, a fastening member may be provided in the coupling portion 10122. The fastening member may be fastened to the lower portion of the holder 1011 inserted into the coupling portion 10122 to fix a coupling state between the holder 1011 and the coupling portion 10122.

The holder 1011 once inserted may be disengaged from the coupling portion 10122 for replacement of the cartridge 1012 or filling with a functional material.

To ensure smooth transfer along airflow generated by inhalation, a particle size of the functional material may be less than or equal to 5 µm. In addition, when a particle for delivering other scents or tastes is included in the functional material, the particle size of the functional material may be within the range of 20 µm to 50 µm.

The functional material applicable to the inhaler 10 may be provided as, for example, an item of personal preference, such as nicotine or nicotine salt, or other functional materials that may bring about a positive effect to the human body when inhaled, such as glutathione or caffeine.

The functional material applicable to the inhaler 10 may include a material for flavor or taste. In this case, the functional material may include sugar alcohols such as mannitol, xylitol, and the like. In addition, the functional material may include materials such as menthol, saccharin, and the like that are expected to have a cough suppressant effect in addition to taste. In addition, other flavor oils and the like may be applied to the functional material.

The functional material of the inhaler 10 may include a small amount of sweetener such as menthol, saccharin, sucralose, stevia, and the like added as particles for flavor or taste. In this case, the sweetener may be added in an amount of less than 1 to 5 wt% in nicotine dry powder. In addition, a small amount of materials that have a positive effect on a dry powder property, such as other amino acids and magnesium stearate, may be included.

An excipient may be applied for manufacture of each functional material in a dry powder form. In this case, flavor particles greater than or equal to a determined size may function as a carrier.

The inhaler 10 may include the housing 101 including the first surface A1, the second surface A2 opposite to the first surface A1, and the side surface A3 configured to connect the first surface A1 to the second surface A2, wherein the plurality of air inlets 10111 may be formed in the housing 101 to penetrate the side surface A3 in a vertical direction, the mouthpiece 102 protruding from the first surface A1, and the guide 103 configured to form a path through which air introduced through the air inlets 10111 may move in the interior of the housing 101, wherein, when an inhaling force is applied to the mouthpiece 102, the guide 103 may guide a functional material accommodated in the interior of the housing 101 to be transferred to the mouthpiece 102.

The guide 103 may include the first guide 1031 extending from a position adjacent to the air inlets 10111 in a direction from the first surface A1 toward the second surface A2, the second guide 1032 extending from the first guide 1031 toward the second surface A2 in a flange shape, and the third guide 1033 spaced apart from the second guide 1032 and protruding from the second surface A2 in a direction toward the first surface A1, wherein the third guide 1033 may extend in a coaxial direction with the mouthpiece 102.

The third guide 1033 may be formed in a shape in which a cross-sectional area decreases in the direction from the second surface A2 toward the first surface A1.

The third guide 1033 may be formed in a cone shape with a diameter that decreases in the direction from the second surface A2 toward the first surface A1.

The housing 101 may include the holder 1011 connected to the mouthpiece 102 on the first surface A1 and configured to form an internal space extending toward the second surface A2, and the cartridge 1012 including the second surface A2 and detachably coupled to the holder 1011, wherein the cartridge 1012 may accommodate the functional material inside.

The cartridge 1012 may include the accommodation portion 10121 in which the functional material is accommodated, and the coupling portion 10122 spaced apart in an outer radial direction from the accommodation portion 10121 and formed as a groove into which a lower portion of the holder 1011 is inserted, wherein the functional material may move to rise upward in a coaxial direction with the mouthpiece 102 by the guide 103.

The plurality of air inlets 10111 may each be disposed to be spaced apart from another in a circumferential direction of the side surface A3.

The functional material may be manufactured in a powder form.

The inhaler 10 may include the housing 101 including the first surface A1, the second surface A2 opposite to the first surface A1, and the side surface A3 configured to connect the first surface A1 to the second surface A2, wherein a functional material is accommodated in the interior of the housing, the mouthpiece 102 protruding from the first surface A1, and the airflow path 104 formed in the interior of the housing 101 and extending from the plurality of air inlets 10111, which may be configured to penetrate the side surface A3 in a vertical direction, to the mouthpiece 102, wherein, when an inhaling force is applied to the mouthpiece 102, the functional material may be transferred to the mouthpiece 102 along the airflow path 104 together with air introduced through the air inlets 10111.

The airflow path 104 may include the first path 1041 extending toward the second surface A2 in a direction perpendicular to the air inlets 10111, the second path 1042 extending from the first path 1041 to the bottom surface A4 on which the functional material is accommodated, the third path 1043 extending diagonally from the second path 1042 toward an axis of the mouthpiece 102, and the fourth path 1044 extending coaxially with the mouthpiece 102 from the third path 1043 toward the first surface A1, wherein at least a portion of the second path 1042 may include a curved path.

While the embodiments of the present disclosure have been described above with reference to specific components, and limited embodiments and drawings, the above descriptions are merely for better understanding of the present disclosure, and it will be apparent to one of ordinary skill in the art that various changes in form and details may be made in these embodiments without departing from the spirit and scope of the claims and their equivalents. For example, suitable results may be achieved if the described techniques are performed in a different order, and/or if components in a described architecture, device, and the like are combined in a different manner and/or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
a housing comprising a first surface, a second surface opposite to the first surface, and a side surface configured to connect the first surface to the second surface, wherein a plurality of air inlets is formed in the housing to penetrate the side surface in a vertical direction;
a mouthpiece protruding from the first surface; and
a guide configured to form a path through which air introduced through the air inlets moves in an interior of the housing,
wherein, when an inhaling force is applied to the mouthpiece, the guide is configured to guide a functional material accommodated in the interior of the housing to be transferred to the mouthpiece.

2. The inhaler of claim 1, wherein the guide comprises:
a first guide extending from a position adjacent to the air inlets in a direction from the first surface toward the second surface;
a second guide extending from the first guide toward the second surface in a flange shape; and
a third guide disposed to be spaced apart from the second guide and protruding from the second surface in a direction toward the first surface,
wherein the third guide extends in a coaxial direction with the mouthpiece.

3. The inhaler of claim 2, wherein the third guide is formed in a shape in which a cross-sectional area decreases in the direction from the second surface to the first surface.

4. The inhaler of claim 3, wherein the third guide is formed in a cone shape with a diameter that decreases in the direction from the second surface to the first surface.

5. The inhaler of claim 1, wherein the housing comprises:
a holder connected to the mouthpiece on the first surface and configured to form an internal space extending toward the second surface; and
a cartridge comprising the second surface and detachably coupled to the holder,
wherein the cartridge accommodates the functional material inside.

6. The inhaler of claim 5, wherein the cartridge comprises:
an accommodation portion in which the functional material is accommodated; and
a coupling portion spaced apart in an outer radial direction from the accommodation portion and formed as a groove into which a lower portion of the holder is inserted,
wherein the functional material moves to rise upward in a coaxial direction with the mouthpiece by the guide.

7. The inhaler of claim 1, wherein each of the plurality of air inlets is disposed to be spaced apart from another in a circumferential direction of the side surface.

8. The inhaler of claim 1, wherein the functional material is manufactured in a powder form.

9. An inhaler comprising:
a housing comprising a first surface, a second surface opposite to the first surface, and a side surface configured to connect the first surface to the second surface, wherein a functional material is accommodated in an interior of the housing;
a mouthpiece protruding from the first surface; and
an airflow path formed in the interior of the housing and extending from a plurality of air inlets, which is configured to penetrate the side surface in a vertical direction, to the mouthpiece,
wherein, when an inhaling force is applied to the mouthpiece, the functional material is transferred to the mouthpiece along the airflow path together with air introduced through the air inlets.

10. The inhaler of claim 9, wherein the airflow path comprises:
a first path extending toward the second surface in a direction perpendicular to the air inlets;
a second path extending from the first path to a bottom surface of the housing, on which the functional material is accommodated;
a third path extending diagonally from the second path toward an axis of the mouthpiece; and
a fourth path extending coaxially with the mouthpiece from the third path toward the first surface,
wherein at least a portion of the second path comprises a curved path.
